# EUROPÄISCHE PATENTSCHRIFT

(11) **EP 0 763 533 B1**
(45) Veröffentlichungstag und Bekanntmachung des Hinweises auf die Patenterteilung: **13.06.2001**
(21) Anmeldenummer: 96114417.7
(22) Anmeldetag: 10.09.1996
(51) Int. Cl.: C07D 339/04, C07B 53/00, C07C 309/73, C07C 309/66, C07C 303/28, C07C 69/63, C07C 67/307, C07C 319/10, C07B 57/00, C07C 59/115, C07C 69/675, C07C 53/19, C07C 323/52

(54) **Herstellung und Verwendung der reinen Enantiomere der 8-Chlor-6-Sulfonyloxyoctansäure und ihrer Alkylester und der reinen Enantiomere der 6,8-Dichloroctansäure und ihrer Alkylester**
Preparation and use of the pure enantiomers of 8-chloro-6-sulfonyloxy-octanoic acid and its alkyl esters and of the pure enantiomers of 6,8-dichloro-octanoic acid and its alkyl esters
Préparation et utilisation des énantiomères purs d'acide 8-chloro-6-sulfonyloxy-octanoique et ses esters d'alkyle et des énantiomères purs d'acide 6,8-dichloro-octanoique et ses esters d'alkyle

(30) Priorität: 13.09.1995 DE 19533881
(43) Veröffentlichungstag der Anmeldung: 19.03.1997
(73) Patentinhaber: Arzneimittelwerk Dresden GmbH, 01445 Radebeul (DE)
(72) Erfinder: Gewald, Rainer, Dr., 01217 Dresden (DE); Laban, Gunter, Dr., 01465 Langebrück (DE); Beisswenger, Thomas, Dr., 01445 Radebeul (DE)
(74) Vertreter: Wibbelmann, Jobst, Dr., Dipl.-Chem.

(56) Entgegenhaltungen:
- EP-A- 0 372 816
- FR-A- 1 176 920
- GB-A- 933 809
- US-A- 2 792 406
- US-A- 2 877 235
- US-A- 2 980 716
- J. AM. CHEM. SOC., Bd. 79, 1957, Seiten 6483-6487, XP002022470 D.S. ACKER ET AL:
- J. AM. CHEM. SOC., Bd. 77, 1955, Seiten 5144-5149, XP002022471 E. WALTON ET AL:
- "HOUBEN-WEYL Methoden der Organischen Chemie, Band E11" 1985 , GEORGE THIEME VERLAG , STUTTGART, DE XP002022472 * Seite 55 *

## Beschreibung

Die Erfindung betrifft Verfahren zur Herstellung der enantiomerenreinen α-Liponsäuren der Formel IV sowie der enantiomerenreinen Dihydroliponsäuren der Formel V aus den reinen Enantiomeren der 8-Chlor-6-hydroxy-octansäure der Formel VI. Weiterhin betrifft die Erfindung neue (+)- und (-)-8-Chlor-6-sulfonyloxyoctansäuren der Formel I und deren enantiomerenreine Ester der Formel II sowie neue (+)- und (-)-6,8-Dichlor-octansäureester der Formel III sowie Verfahren zu deren Herstellung und zur Herstellung der enantiomerenreinen α-Liponsäuren der Formel IV sowie der enantiomerenreinen Dihydroliponsäuren der Formel V. α-Liponsäure ist 1,2-Dithiolan-3-pentansäure (Thioctsäure).

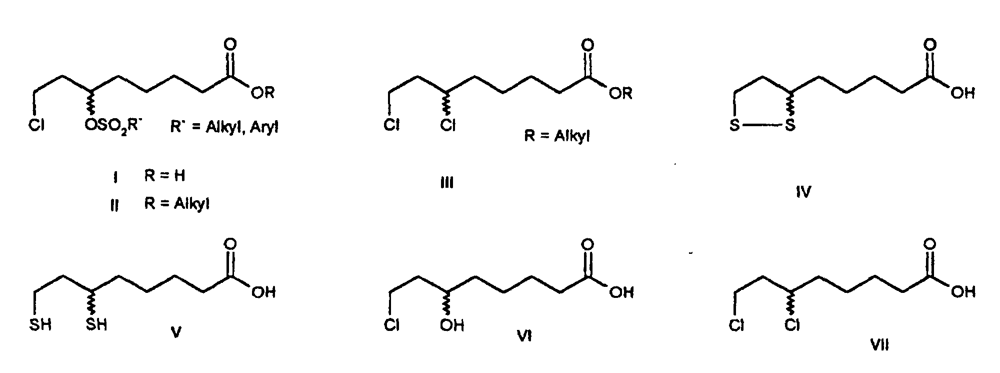

Das R-Enantiomer der α-Liponsäure ist ein Naturstoff, der in geringen Konzentrationen in praktisch allen tierischen und pflanzlichen Zellen vorkommt. Als Coenzym bei der oxidativen Decarboxylierung von α-Ketocarbonsäuren (z.B. Brenztraubensäure) ist α-Liponsäure von essentieller Bedeutung. α-Liponsäure ist pharmakologisch wirksam und weist antiphlogistische und antinociceptive (analgetische) sowie zytoprotektive Eigenschaften auf. Eine wichtige medizinische Indikation ist die Behandlung der diabetischen Polyneuropathie. Nach neueren Ergebnissen (vgl. CA 116: 207360) kann α-Liponsäure möglicherweise Bedeutung bei der Bekämpfung durch HIV-1- und HTLV IIIB-Viren bedingter Krankheiten erlangen.

Die Synthese der razemischen Verbindungen der Formel III ist bekannt (US-A-2 792 406; US-A-2 980 716). Die Herstellung der Razemate der Formel II ist ebenfalls beschrieben (US-A-2 877 235),

Bei den reinen optischen Isomeren der α-Liponsäure (R- und S-Form, d.h. R-α-Liponsäure und S-α-Liponsäure) ist im Gegensatz zu dem Razemat das R-Enantiomer vorwiegend antiphlogistisch und das S-Enantiomer vorwiegend antinociceptiv wirksam (EP 0427247, 08.11.90). Daher ist die Synthese der reinen Enantiomere, insbesondere der R-Form, von großer Wichtigkeit.

Bekannte Herstellungsverfahren der enantiomerenreinen α-Liponsäure umfassen die Razematspaltung der α-Liponsäure oder ihrer Vorstufen, asymmetrische Synthesen unter Einsatz chiraler Auxilarien , "chiral pool"-Synthesen unter Verwendung von in der Natur vorkommenden optisch aktiven Ausgangsverbindungen sowie mikrobielle Synthesen (Übersichtsartikel: J.S. Yadav et al., J. Sci. Ind. Res. 1990, 49, 400; sowie: A.G. Tolstikov et al., Bioorg. Khim. 1990, 16, 1670; L. Dasaradhi et al., J. Chem. Soc., Chem. Commun. 1990, 729; A.S. Gopalan et al., J. Chem. Perkin Trans. 1 1990, 1897; A.S. Gopalan et al., Tetrahedron Lett. 1989, 5705; EP 0487986 A2, 14.11.91).

Davon stellt die Razematspaltung über die Bildung von diastereomeren Salzen der α-Liponsäure mit optisch aktivem α-Methylbenzylamin ( DE-OS 4137773.7, 16.11.91) die bisher wirtschaftlichste Variante dar. Der Nachteil dieses Verfahrens besteht jedoch darin, daß die Razemattrennung erst auf der letzten Stufe der Synthesesequenz erfolgt und das unerwünschte Enantiomer der α-Liponsäure weder razemisiert noch invertiert werden kann. Auch bei den anderen bekannten Razematspaltungsverfahren auf Vorstufen der α-Liponsäure kann jeweils nur ein Enantiomer in das gewünschte optische Isomer der α-Liponsäure überführt und somit nur eine theoretische Ausbeute von 50% erreicht werden (E. Walton et al. J. Am. Chem. Soc. 1955, 77, 5144; D.S. Acker und W.J. Wayne, J. Am. Chem. Soc. 1957, 79, 6483; L.G. Chebotareva und A.M. Yurkevich, Khim.-Farm. Zh. 1980, 14, 92).

Aufgabe der Erfindung ist es deshalb, ein Verfahren zur Herstellung der enantiomerenreinen a-Liponsäuren und Dihydroliponsäuren zu schaffen, bei dem die Razematspaltung auf der frühestmöglichen Stufe der Synthesesequenz durchgeführt wird, wobei die Umwandlung beider anfallender Enantiomere der 8-Chlor-6-hydroxy-octansäure in ein Enantiomer der α-Liponsäure mit einer theoretischen Ausbeute von 100% ohne zusätzliche Razemisierungs- oder Inversionsschritte möglich ist.

In den folgenden beiden Übersichten ist der Weg zur Herstellung der R(+)- und S(-)-α-Liponsäure schematisch dargestellt, wobei R vorzugsweise eine geradkettige oder verzweigte C₁-C₄-Alkylgruppe, d.h. eine Methyl-, Ethyl-, n-Propyl-, iso-Propyl-, n-Butyl,-, iso-Butyl- oder tert-Butylgruppe, insbesondere eine Methylgruppe, bedeutet und R'vorzugsweise eine Methyl- oder p-Tolylgruppe bedeutet.

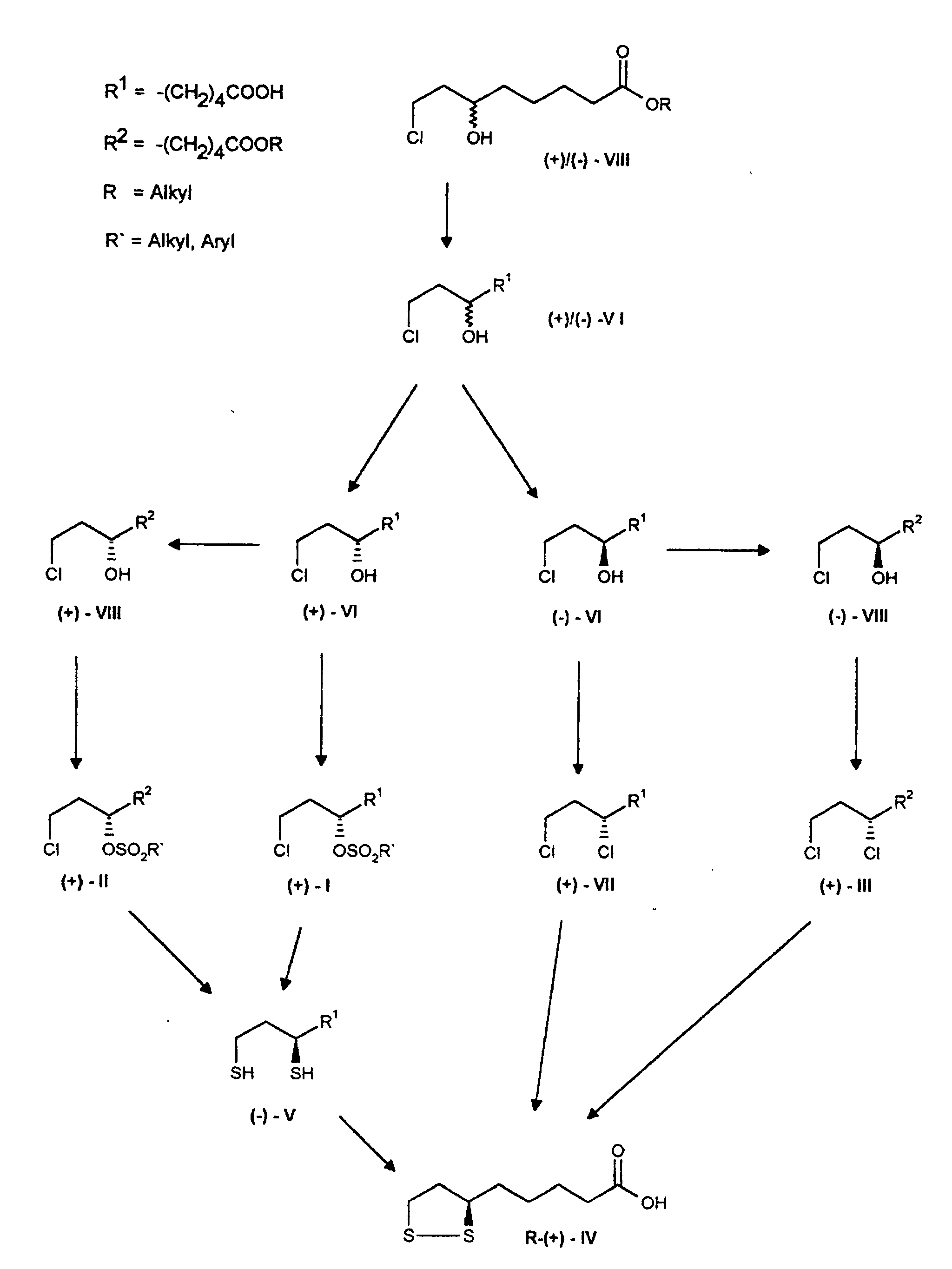

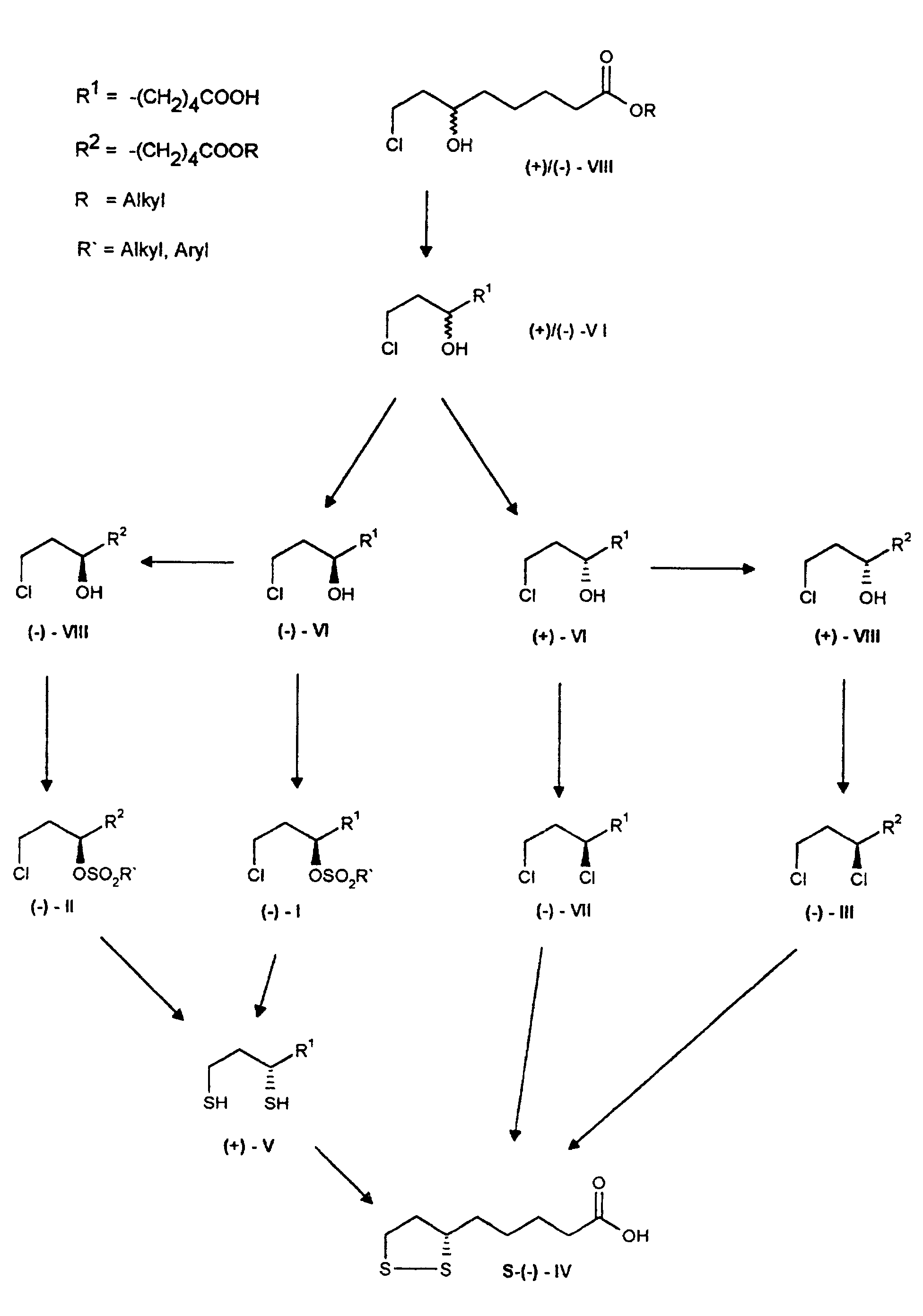

Das Ausgangsmaterial des Verfahrens, die razemische 8-Chlor-6-hydroxyoctansäure der Formel VI, wird auf bekanntem Wege durch Hydrolyse ihrer razemischen Alkylester der Formel VIII gewonnen (Y. Deguchi und K. Nakanishi, Yakugaku Zasshi 1963, 83, 701).

Die reine (+)-8-Chlor-6-hydroxy-octansäure oder die reine (-)-8-Chlor-6-hydroxy-octansäure können durch Umsetzung der razemischen 8-Chlor-6-hydroxyoctansäure mit den optischen Antipoden des α-Methylbenzylamins, Bildung der diastereomeren Salzpaare und Isolierung des schwerer löslichen Salzes mit anschließender Spaltung der reinen diastereomeren Salze aus (+)-8-Chlor-6-hydroxy-octansäure und R-(+)-α-Methylbenzylamin beziehungsweise (-)-8-Chlor-6-hydroxy-octansäure und S-(-)-α-Methylbenzylamin unter Zusatz von Säuren, z.B. Mineralsäuren, oder Basen, z.B. Alkalihydroxyden, gewonnen werden.

Beide Enantiomere der 8-Chlor-6-hydroxy-octansäure der Formel VI können erfindungsgemäß direkt in R-α-Liponsäure der Formel (+)-IV überführt werden, indem man die (+)-8-Chlor-6-hydroxy-octansäure bzw. deren Alkylester unter Erhalt der Konfiguration mit einem Sulfonsäurechlorid umsetzt und die (-)-8-Chlor-6-hydroxy-octansäure bzw. deren Alkylester unter Umkehr der Konfiguration chloriert. Die darauf folgende Schwefeleinführung führt letztendlich von allen Zwischenprodukten aus zur R-α-Liponsäure in vorzüglicher optischer Reinheit (e.e.> 99%, chirale HPLC).

Die Enantiomere der 8-Chlor-6-hydroxy-octansäure der Formel VI lassen sich stereospezifisch unter Erhalt der Konfiguration in Gegenwart von katalytischen Mengen HCI in ihre Alkylester der Formel VIII, vorzugsweise Methylester, umwandeln.

Die enantiomerenreinen (+)-8-Chlor-6-hydroxy-octansäurealkylester der Formel (+)-VIII werden dann unter Erhalt der Konfiguration in die (+)-8-Chlor-6-sulfonyloxy-octansäurealkylester der Formel (+)-II und diese in (-)-Dihydroliponsäure der Formel (-)-I oder R-α-Liponsäure der Formel (+)-IV überführt.

Man kann jedoch von der (+)-8-Chlor-6-hydroxy-octansäure der Formel (+)-VI auch direkt über die (+)-8-Chlor-6-sulfonyloxy-octansäuren der Formel (+)-I in guten Ausbeuten stereospezifisch zur (-)-Dihydroliponsäure der Formel (-)-V oder R-α-Liponsäure der Formel (+)-IV gelangen. Die Umsetzung mit dem Sulfonsäurechlorid wird dann unter Einsatz von 1,5 bis 3, vorzugsweise jedoch 2,0 bis 2,2 Moläquivalenten an Sulfonsäurechlorid und 1,5 bis 2,5, vorzugsweise jedoch 2,0 bis 2,1 Moläquivalenten an tertiärer Stickstoffbase, bevorzugt Triethylamin, durchgeführt.

Die Umwandlung der enantiomerenreinen Dihydroliponsäuren der Formel V in die optischen Isomere der α-Liponsäure der Formel IV durch Oxydation mit Luft in Gegenwart katalytischer Mengen von Eisen(III)-Salzen ist aus der Literatur bekannt (E. Walton et al. J. Am. Chem. Soc. 1955, 77, 5144).

Die enantiomerenreinen (-)-8-Chlor-6-hydroxy-octansäurealkylester der Formel (-)-VIII werden unter Konfigurationsumkehr durch Umsetzung mit Thionylchlorid in Gegenwart katalytischer Mengen Pyridin in die (+)-6,8-Dichloroctansäurealkylester der Formel (+)-VII überführt. Die darauf folgende Schwefeleinführung mit Na₂S₂ ergab R-α-Liponsäure der Formel (+)-IV in hoher Enantiomerenreinheit.

Vorteilhafterweise kann auch die (-)-8-Chlor-6-hydroxy-octansäure der Formel (-)-VI direkt in guten Ausbeuten in die (+)-6,8-Dichlor-octansäure der Formel (+)-VII umgewandelt werden, indem im Chlorierungsschritt 1,5 bis 5, vorzugsweise jedoch 2,0 bis 2,5 Moläquivalente Thionylchlorid eingesetzt werden und das Reaktionsgemisch in einem Eintopfverfahren durch Zugabe von wäßrigen Basen, vorzugsweise Natronlauge, hydrolytisch aufgearbeitet wird. Die weitere Umsetzung der (+)-6,8-Dichlor-octansäure mit Na₂S₂ zur R-α-Liponsäure der Formel (+)-IV ist aus der Literatur bekannt (D.S. Acker und W.J. Wayne, J. Am. Chem. Soc. 1957, 79, 6483).

Durch Umsetzung der (-)-8-Chlor-6-hydroxy-octansäure der Formel (-)-VI bzw. deren Alkylester der Formel (-)-VIII mit Sulfonsäurechloriden und Chlorierung der (+)-8-Chlor-6-hydroxy-octansäure der Formel (+)-VI bzw. deren Alkylester der Formel (+)-VIII läßt sich erfindungsgemäß analog dem oben beschriebenem Verfahren S-α-Liponsäure der Formel S-(-)-IV erhalten.

Zur Herstellung der enatiomerenreinen (+)- bzw. (-)-Dihydroliponsäure kann man auch S(-)-α-Liponsäure bzw. R(+)-α-Liponsäure in an sich bekannter Weise reduzieren.

Alle obengenannten Umsetzungen werden bevorzugt in einem geeigneten organischen Lösemittel durchgeführt. Beispiele für organische Lösemittel sind aliphatische Kohlenwasserstoffe mit einer Kohlenstoffkettenlänge zwischen 3 und 10 Kohlenstoffatomen, aromatische Kohlenwasserstoffe, die flüssig sind, Ester aus aliphatischen oder cycloaliphatischen Carbonsäuren mit 2 bis 6 Kohlenstoffatomen und aliphatischen oder cycloaliphatischen Alkoholen mit 2 bis 6 Kohlenstoffatomen, aliphatischen oder cycloaliphatischen Alkoholen mit 1 bis 6 Kohlenstoffatomen, Ether und Glycolether oder homogene Gemische der genannten Lösungsmittel. Besonders bevorzugte Lösemittel sind Essigsäureethylester, Cyclohexan, Toluol, Ethanol und deren homogene Gemische.

Die Reinheit der optischen Isomere und der diastereomeren Salze wurde mittels der spezifischen optischen Drehwerte bestimmt. Weiterhin wurden die relativen Gehalte der optischen Isomere der 8-Chlor-6-hydroxy-octansäure der Formel VI und der α-Liponsäure der Formel IV durch HPLC an optisch aktiven Säulen mit einer Nachweisgrenze von 0,5% ermittelt. Zusätzlich erfolgte die Bestimmung der optischen Reinheit der 8-Chlor-6-hydroxy-octansäurealkylester der Formel VIII durch ¹H-NMR-Analyse der Ester, die bei der Reaktion mit (S)-(+)-O-Acetylmandelsäure gebildet werden.

Die vorliegende Erfindung ermöglicht es, die Enantiomeren der a-Liponsäure auf einfache und wirtschaftliche Weise in hoher chemischer und optischer Ausbeute zugänglich zu machen.

Sie wird durch nachfolgende Beispiele näher erläutert.

### Beispiel 1

39,9 g (204 mmol) razemische 8-Chlor-6-hydroxy-octansäure (+)/(-)-VI wurden bei 40°C in 155 ml eines Gemisches aus Ethylacetat und Cyclohexan (1:1) aufgelöst. Innerhalb von 10 min wurden 13,5 g (112 mmol) R-(+)-α-Methylbenzylamin zudosiert. Anschließend wurde über einen Zeitraum von 2 h auf 20°C abgekühlt, filtriert und der Niederschlag mit 20 ml Lösungsmittelgemisch Ethylacetat/Cyclohexan (1:1) und 30 ml Cyclohexan nachgewaschen. Das Salz wurde zweimal aus 400 ml Ethylacetat/Cyclohexan (3:1) umkristallisiert und im Vakuum bei 40°C getrocknet. Man erhielt 20,5 g (+)(+)-Diastereomerensalz, α = +22,7° (c = 1; Ethanol).

Das Salz wurde bei 20°C in 220 ml Diethylether suspendiert. Unter Eiskühlung und Rühren wurde mit 3 N Salzsäure langsam auf einen pH-Wert von 1 eingestellt, wobei das Salz in Lösung geht. Nach weiteren 30 min wurden die Phasen getrennt und die organische Phase einmal mit 20 ml 2 N HCI und zweimal mit 20 ml Wasser gewaschen und über Magnesiumsulfat getrocknet. Nach Abziehen des Lösemittels im Vakuum wurden 10,8 g (54% d. Th.) (+)-8-Chlor-6-hydroxy-octansäure (+)-VI erhalten; [α]_{D}²⁰ = +24,5° (c = 1; Ethanol), e.e.: >99% (HPLC), Schmelzpunkt 29-30°G.

### Beispiel 2

33,9 g (173 mmol) razemische 8-Chlor-6-hydroxy-octansäure (+)/(-)-VI wurden bei 40°C in 130 ml eines Gemisches aus Ethylacetat und Cyclohexan (1:1) aufgeöst. Innerhalb von 10 min wurden 11,5 g (95 mmol) S-(-)-α-Methylbenzylamin zudosiert. Anschließend wurde über einen Zeitraum von 2 h auf 20°C abgekühlt, filtriert und der Niederschlag mit 17 ml Lösungsmittelgemisch Ethylacetat/Cyclohexan (1:1) und 25 ml Cyclohexan nachgewaschen. Das Salz wurde zweimal aus 340 ml Ethylacetat/Cyclohexan (3:1) umkristallisiert und im Vakuum bei 40°C getrocknet. Man erhielt 17,2 g (-)(-)-Diastereomerensalz, α = -22,7° (c = 1; Ethanol).

Das Salz wurde bei 20°C in 190 ml Diethylether suspendiert. Unter Eiskühlung und Rühren wurde mit 3 N Salzsäure langsam auf einen pH-Wert von 1 eingestellt, wobei das Salz in Lösung geht. Nach weiteren 30 min wurden die Phasen getrennt und die organische Phase einmal mit 17 ml 2 N HCI und zweimal mit 20 ml Wasser gewaschen und über Magnesiumsulfat getrocknet. Nach Abziehen des Lösemittels im Vakuum wurden 9,1 g (53% d. Th.) (-)-8-Chlor-6-hydroxy-octansäure (-)-VI erhalten;[α]_{D}²⁰ = -24,5° (c = 1; Ethanol), e.e.: >99% (HPLC), Schmelzpunkt 29-30°C.

### Beispiel 3

6,4- g (32,9 mmol) (+)-8-Chlor-6-hydroxyoctansäure (+)-VI wurden in 100 ml absolutem Methanol nach Zugabe von 0,4 ml konzentrierter Salzsäure 2 h am Rückfluß erhitzt. Danach wurde das Lösemittel im Vakuum abgezogen. Man erhielt 6,6 g (97% d. Th.) (+)-8-Chlor-6-hydroxy-octansäuremethylester (+)-VIII (R=Me), [α]_{D}²⁰ = +24,5° (c = 1; Ethanol), e.e.: >99% (¹H NMR).

### Beispiel 4

7,7 g (39,5 mmol) (-)-8-Chlor-6-hydroxy-octansäure (-)-VI wurden in 120 ml absolutem Methanol nach Zugabe von 0,5 ml konzentrierter Salzsäure 2 h am Rückfluß erhitzt. Danach wurde das Lösemittel im Vakuum abgezogen. Man erhielt 7,9 g (97% d. Th.) (-)-8-Chlor-6-hydroxy-octansäuremethylester (-)-VIII (R=Me), [α]_{D}²⁰ = -24,5° (c = 1; Ethanol), e.e.: >99% (¹H NMR).

### Beispiel 5

3,9 g (20 mmol) (+)-8-Chlor-6-hydroxy-octansäure (+)-VI und 4,1 g (40 mmol) Triethylamin wurden in 80 ml Toluol gemischt. Dazu wurden unter Kühlung (Innentemperatur 10-15°C) langsam 3,5 g (30,6 mmol) Methansulfonsäurechlorid gegeben. Es wurde 30 min nachgerührt. Nach Zusatz von 25 ml Wasser wurde nochmals 30 min gerührt, danach die organische Phase abgetrennt und über Magnesiumsulfat getrocknet. Danach wurde das Lösemittel im Vakuum abgezogen. Man erhielt 3,8 g (69% d. Th.) (+)-8-Chlor-6-methansulfonyloxy-octansäure (+)-I (R'=Me), [α]_{D}²⁰ = +32,9° (c = 1; Ethanol).

### Beispiel 6

6,6 g (34 mmol) (-)-8-Chlor-6-hydroxy-octansäure (-)-VI und 7,0 g (68 mmol) Triethylamin wurden in 140 ml Toluol gemischt. Dazu wurden unter Kühlung (Innentemperatur 10-15°C) langsam 6,0 g (52,6 mmol) Methansulfonsäurechlorid gegeben. Es wurde 30 min nachgerührt. Nach Zusatz von 40 ml Wasser wurde nochmals 30 min gerührt, danach die organische Phase abgetrennt und über Magnesiumsulfat getrocknet. Danach wurde das Lösemittel im Vakuum abgezogen. Man erhielt 6,5 g (70% d. Th.) (-)-8-Chlor-6-methansulfonyloxy-octansäure (-)-I (R'=Me), [α]_{D}²⁰ = -32,8° (c = 1; Ethanol).

### Beispiel 7

4,0 g (19,2 mmol) (+)-8-Chlor-6-hydroxy-octansäuremethylester (+)-VIII (R=Me) und 1,97 g (19,2 mmol) Triethylamin wurden in 90 ml Toluol gemischt. Dazu wurden unter Kühlung (Innentemperatur 10-15°C) langsam 2,63 g (23,0 mmol) Methansulfonsäurechlorid gegeben. Es wurde 30 min nachgerührt. Nach Zusatz von 30 ml Wasser wurde nochmals 30 min gerührt, danach die organische Phase abgetrennt und über Magnesiumsulfat getrocknet. Danach wurde das Lösemittel im Vakuum abgezogen. Man erhielt 4,8 g (88% d. Th.) (+)-8-Chlor-6-methansulfonyloxy-octansäuremethylester (+)-II (R=R'=Me), [[α]_{D}²⁰ = +31,2° (c = 1; Ethanol).

### Beispiel 8

2,1 g (10 mmol) (-)-8-Chlor-6-hydroxy-octansäuremethylester (-)-VIII (R=Me) und 1,0 g (10 mmol) Triethylamin wurden in 40 ml Toluol gemischt. Dazu wurden unter Kühlung (Innentemperatur 10-15°C) langsam 1,4 g (12 mmol) Methansulfonsäurechlorid gegeben. Es wurde 30 min nachgerührt. Nach Zusatz von 25 ml Wasser wurde nochmals 30 min gerührt, danach die organische Phase abgetrennt und über Magnesiumsulfat getrocknet. Danach wurde das Lösemittel im Vakuum abgezogen. Man erhielt 2,5 g (86% d. Th.) (-)-8-Chlor-6-methansulfonyloxyoctansäuremethylester (-)-II (R=R'=Me), [α]_{D}²⁰ = -31,3° (c = 1; Ethanol).

### Beispiel 9

Zu einer Lösung aus 2,4 g (11,0 mmol) (+)-8-Chlor-6-hydroxyoctansäuremethylester (+)-VIII (R=Me) und 0,04 g (0,5 mmol) Pyridin in 8 ml Toluol wurden langsam 1,6 g (13,5 mmol) Thionylchlorid in 5 ml Toluol gegeben. Dann wurde 1 h am Rückfluß erhitzt. Nach Abkühlung auf Raumtemperatur wurde das Reaktionsgemisch in 20 ml Eiswasser gegeben, die organische Phase abgetrennt, mit 10 ml Wasser gewaschen und über Magnesiumsulfat getrocknet. Danach wurde das Lösemittel im Vakuum abgezogen. Man erhielt 2,0 g (81% d. Th.) (-)-6,8-Dichlor-octansäuremethylester (-)-III (R=Me), [α]_{D}²⁰ = -30,0° (c = 1; Benzol).

### Beispiel 10

Zu einer Lösung aus 2,9 g (13,2 mmol) (-)-8-Chlor-6-hydroxyoctansäuremethylester (-)-VIII (R=Me) und 0,05 g (0,6 mmol) Pyridin in 10 ml Toluol wurden langsam 1,9 g (16,2 mmol) Thionylchlorid in 6 ml Toluol gegeben. Dann wurde 1 h am Rückfluß erhitzt. Nach Abkühlung auf Raumtemperatur wurde das Reaktionsgemisch in 25 ml Eiswasser gegeben, die organische Phase abgetrennt, mit 10 ml Wasser gewaschen und über Magnesiumsulfat getrocknet. Danach wurde das Lösemittel im Vakuum abgezogen. Man erhielt 2,4 g (81% d. Th.) (+)-6,8-Dichlor-octansäuremethylester (+)-III (R=Me), [α]_{D}²⁰ = +30,1° (c = 1; Benzol).

### Beispiel 11

Zu einer Lösung aus 2,4 g (12,3 mmol) (+)-8-Chlor-6-hydroxy-octansäure (+)-VI und 0,05 g (0,6 mmol) Pyridin in 30 ml Toluol wurden langsam 3,3 g (27,7 mmol) Thionylchlorid gegeben. Dann wurde 1 h am Rückfluß erhitzt. Nach Abkühlung auf Raumtemperatur wurde das Reaktionsgemisch in 50 ml Eiswasser gegeben, die organische Phase abgetrennt, mit 20 ml Wasser gewaschen und 4 h mit 30 ml 2 N NaOH gerührt. Die wäßrige Phase wurde dann abgetrennt, mit 3 N HCI auf pH=1 eingestellt und mit zweimal 20 ml Diethylether extrahiert. Die vereinigten etherischen Extrakte wurden über Magnesiumsulfat getrocknet. Danach wurde das Lösemittel im Vakuum abgezogen. Man erhielt 2,1 g (80% d. Th.) (-)-6,8-Dichlor-octansäure (-)-VII, [α]_{D}²⁰ = -30,6° (c = 1; Benzol).

### Beispiel 12

Zu einer Lösung aus 3,0 g (15,4 mmol) (-)-8-Chlor-6-hydroxy-octansäure (-)-VI und 0,06 g (0,8 mmol) Pyridin in 40 ml Toluol wurden langsam 4,1 g (34,4 mmol) Thionylchlorid gegeben. Dann wurde 1 h am Rückfluß erhitzt. Nach Abkühlung auf Raumtemperatur wurde das Reaktionsgemisch in 60 ml Eiswasser gegeben, die organische Phase abgetrennt, mit 20 ml Wasser gewaschen und 4 h mit 35 ml 2 N NaOH gerührt. Die wäßrige Phase wurde dann abgetrennt, mit 3 N HCl auf pH=1 eingestellt und mit zweimal 20 ml Diethylether extrahiert. Die vereinigten etherischen Extrakte wurden über Magnesiumsulfat getrocknet. Danach wurde das Lösemittel im Vakuum abgezogen. Man erhielt 2,6 g (80% d. Th.) (+)-6,8-Dichlor-octansäure (+)-VII, [α]_{D}²⁰ = +30,5° (c = 1; Benzol).

### Beispiel 13

Ein Gemisch aus 4,6 g (19 mmol) Natriumsulfid-Nonahydrat und 0,61 g (19 mmol) Schwefel wurde in 40 ml Ethanol 15 min unter Rückfluß erhitzt. Dazu wurde über einen Zeitraum von 2 h bei 20°C eine Lösung aus 4,9 g (17 mmol) (+)-8-Chlor-6-methansulfonyloxy-octansäuremethylester (+)-II (R=R'=Me) und 5 ml Ethanol gegeben und 3 h nachgerührt. Dann wurden 24 ml 10%ige NaOH zugegeben und 2 h bei 25°C gerührt. Nach Abziehen des Ethanols im Vakuum wurde bei 25°C eine Lösung aus 0,37 g (9,7 mmol) Natriumborhydrid in 10 ml 1%iger NaOH innerhalb 10 min zum Reaktionsgemisch gegeben und unter Rühren langsam auf 100°C erwärmt und 1 h bei dieser Temperatur gerührt. Nach Abkühlen wurde das Reaktionsgemisch mit konz. HCI auf pH 1 angesäuert und zweimal mit 20 ml Diethylether extrahiert. Die organischen Phasen wurden über Natriumsulfat getrocknet und das Lösemittel im Vakuum abgezogen. Man erhielt 2,8 g (79% d. Th.) (-)- Dihydroliponsäure (-)-V, [α]_{D}²⁰ = -13,7° (c = 1,5; Ethanol).

### Beispiel 14

Ein Gemisch aus 3,1 g (13 mmol) Natriumsulfid-Nonahydrat und 0,41 g (13 mmol) Schwefel wurde in 25 ml Ethanol 15 min unter Rückfluß erhitzt. Dazu wurde über einen Zeitraum von 2 h bei 20°C eine Lösung aus 3,3 g (11 mmol) (-)-8-Chlor-6-mathansulfonyoxy-octansäuremethylester (-)-II (R=R'=Me) und 5 ml Ethanol gegeben und 3 h nachgerührt. Dann wurden 15 ml 10%ige NaOH zugegeben und 2 h bei 25°C gerührt. Nach Abziehen des Ethanols im Vakuum wurde bei 25°C eine Lösung aus 0,25 g (6,6 mmol) Natriumborhydrid in 10 ml 1%iger NaOH innerhalb 10 min zum Reaktionsgemisch gegeben und unter Rühren langsam auf 100°C erwärmt und 1 h bei dieser Temperatur gerührt. Nach Abkühlen wurde das Reaktionsgemisch mit konz. HCI auf pH 1 angesäuert und zweimal mit 15 ml Diethylether extrahiert. Die organischen Phasen wurden über Natriumsulfat getrocknet und das Lösemittel im Vakuum abgezogen. Man erhielt 1,9 g (83% d. Th.) (+)- Dihydroliponsäure (+)-V, [α]_{D}²⁰ = +13,7° (c = 1,5; Ethanol).

### Beispiel 15

Ein Gemisch aus 1,5 g (6,3 mmol) Natriumsulfid-Nonahydrat und 0,2 g (6,3 mmol) Schwefel wurde in 15 ml Ethanol 15 min unter Rückfluß erhitzt. Dazu wurde über einen Zeitraum von 2 h bei 20°C eine Lösung aus 1,5 g (5,5 mmol) (+)-8-Chlor-6-methansulfonyloxy-octansäure (+)-I und 4 ml Ethanol gegeben und 3 h nachgerührt. Dann wurden 15 ml 10%ige NaOH zugegeben und 2 h bei 25°C gerührt. Nach Abziehen des Ethanols im Vakuum wurde bei 25°C eine Lösung aus 0,12 g (3,2 mmol) Natriumborhydrid in 4 ml 1%iger NaOH innerhalb 10 min zum Reaktionsgemisch gegeben und unter Rühren langsam auf 100°C erwärmt und 1 h bei dieser Temperatur gerührt. Nach Abkühlen wurde das Reaktionsgemisch mit konz. HCI auf pH 1 angesäuert und zweimal mit 10 ml Diethylether extrahiert. Die organischen Phasen wurden über Natriumsulfat getrocknet und das Lösemittel im Vakuum abgezogen. Man erhielt 0,8 g (70% d. Th.) (-)- Dihydroliponsäure (-)-V, [α]_{D}²⁰ = -13,5° (c = 1,0; Ethanol).

### Beispiel 16

Ein Gemisch aus 1,8 g (7,6 mmol) Natriumsulfid-Nonahydrat und 0,24 g (7,6 mmol) Schwefel wurde in 18 ml Ethanol 15 min unter Rückfluß erhitzt. Dazu wurde über einen Zeitraum von 2 h bei 20°C eine Lösung aus 1,8 g (6,6 mmol) (-)-8-Chlor-6-methansulfonyloxy-octansäure (-)-I und 5 ml Ethanol gegeben und 3 h nachgerührt. Dann wurden 18 ml 10%ige NaOH zugegeben und 2 h bei 25°C gerührt. Nach Abziehen des Ethanols im Vakuum wurde bei 25°C eine Lösung aus 0,14 g (3,8 mmol) Natriumborhydrid in 5 ml 1%iger NaOH innerhalb 10 min zum Reaktionsgemisch gegeben und unter Rühren langsam auf 100°C erwärmt und 1 h bei dieser Temperatur gerührt. Nach Abkühlen wurde das Reaktionsgemisch mit konz. HCI auf pH 1 angesäuert und zweimal mit 10 ml Diethylether extrahiert. Die organischen Phasen wurden über Natriumsulfat getrocknet und das Lösemittel im Vakuum abgezogen. Man erhielt 1,0 g (73% d. Th.) (+)- Dihydroliponsäure (+)-V, [α]_{D}²⁰ = -13,6° (c = 1,0; Ethanol).

### Beispiel 17

Ein Gemisch aus 0,62 g (2,6 mmol) Natriumsulfid-Nonahydrat und 0,08 g (2,6 mmol) Schwefel wurde in 10 ml Ethanol 15 min unter Rückfluß erhitzt. Dazu wurde über einen Zeitraum von 1 h unter schwachem Rückfluß eine Lösung aus 0,55 g (2,4 mmol) (+)-6,8-Dichlor-octansäuremethylester (+)-III (R=Me) und 5 ml Ethanol gegeben. Es wurde 15 min nachgerührt und 8 ml Ethanol abdestilliert. Dann wurden 10 ml 0,5 N NaOH zugegeben und 12 h bei 25°C gerührt. Nach Ansäuern des Reaktionsgemisches mit konz. HCI auf pH 1 wurde zweimal mit 20 ml Diethylether extrahiert, die organischen Phasen über Natriumsulfat getrocknet und das Lösemittel im Vakuum abgezogen. Durch Umkristallisation aus Cyclohexan erhielt man 0,28 g (57% d. Th.) R-(+)-α-Liponsäure (+)-IV, Schmelzpunkt 44-46°C, e.e.:>99% (HPLC).

### Beispiel 18

Ein Gemisch aus 0,87 g (3,6 mmol) Natriumsulfid-Nonahydrat und 0,11 g (3,6 mmol) Schwefel wurde in 15 ml Ethanol 15 min unter Rückfluß erhitzt. Dazu wurde über einen Zeitraum von 1 h unter schwachem Rückfluß eine Lösung aus 0,77 g (3,4 mmol) (-)-6,8-Dichlor-octansäuremethylester (-)-III (R=Me) und 7 ml Ethanol gegeben. Es wurde 15 min nachgerührt und 12 ml Ethanol abdestilliert. Dann wurden 14 ml 0,5 N NaOH zugegeben und 12 h bei 25°C gerührt. Nach Ansäuern des Reaktionsgemisches mit konz. HCI auf pH 1 wurde zweimal mit 20 ml Diethylether extrahiert, die organischen Phasen über Natriumsulfat getrocknet und das Lösemittel im Vakuum abgezogen. Durch Umkristallisation aus Cyclohexan erhielt man 0,38 g (54% d. Th.) S-(-)-α-Liponsäure (-)-IV, Schmelzpunkt 44-46°C, e.e.:>99% (HPLC).

## Patentansprüche

1. (+)- und (-)-8-Chlor-6-sulfonyloxy-octansäuren der Formel 1, 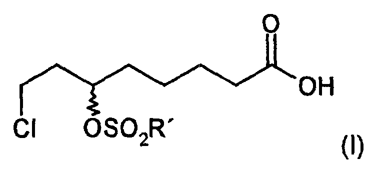 in der R' geradkettige und verzweigte C₁ - C₄-Alkyl- und Arylgruppen bezeichnet.

2. (+)- und (-)-8-Chlor-6-sulfonyloxy-octansäurealkylester der Formel II, 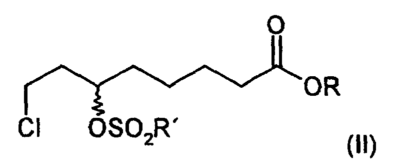 in der R geradkettige und verzweigte C₁ - C₄-Alkylgruppen und R' geradkettige und verzweigte C₁ - C₄-Alkyl- und Arylgruppen bezeichnen.

3. Verfahren zur Herstellung von (+)- und (-)-8-Chlor-6-sulfonyloxy-octansäuren, dadurch gekennzeichnet, daß man (+)- und (-)-8-Chlor-6-hydroxy-octansäure mit 1,5 bis 3 Moläquivalenten Sulfonsäurechlorid und 1,5 bis 2,5 Moläquivalenten tertiärer Stickstoffbase bei 0-30°C in einem organischen Lösemittel umsetzt.

4. Verfahren zur Herstellung von (+)- und (-)-8-Chlor-6-sulfonyloxyoctansäurealkylestern der Formel II, 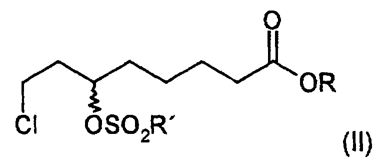 in der R geradkettige und verzweigte C₁ - C₄-Alkylgruppen und R' geradkettige und verzweigte C₁ - C₄-Alkyl- und Arylgruppen bezeichnen, dadurch gekennzeichnet, daß man (+)- und (-)-8-Chlor-6-hydroxy-octansäurealkylester der Formel VIII 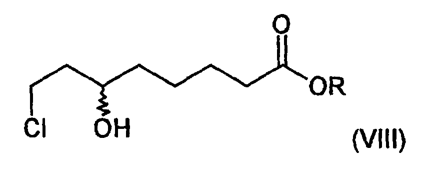 in der R die gleiche Bedeutung wie oben hat, mit 1,0 bis 1,5 Moläquivalenten Sulfonsäurechlorid und 1,0 bis 1,5 Moläquivalenten tertiärer Stickstoffbase bei 0-30°C in einem organischen Lösemittel umsetzt.

5. Verfahren zur Herstellung von R-(+)-α-Liponsäure, dadurch gekennzeichnet, daß man
a) aus (+)-8-Chlor-6-hydroxy-octansäure mit Sulfonsäurechloriden und einer tertiären Stickstoffbase (+)-8-Chlor-6-sulfonyloxy-octansäure der Formel (+)-I 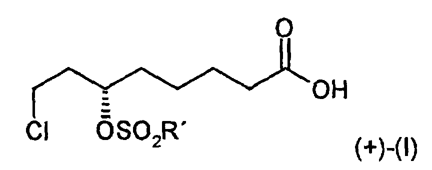 in der R' geradkettige und verzweigte C₁ - C₄-Alkyl- und Arylgruppen bezeichnet, herstellt, diese mit Alkalimetalldisulfid/Schwefel oder Thioharnstoff zur R-(+)-α-Liponsäure oder (-)-Dihydroliponsäure, welche anschließend durch Oxydation in die R-(+)-α-Liponsäure überführt wird, umsetzt oder
b) aus (-)-8-Chlor-6-hydroxy-octansäure mit Thionylchlorid in Gegenwart von Pyridin (+)-6,8-Dichlor-octansäure herstellt und diese mit Alkalimetalldisulfid/Schwefel in die R-(+)-α-Liponsäure überführt.

6. Verfahren zur Herstellung von R-(+)-α-Liponsäure, dadurch gekennzeichnet, daß man
a) (+)-8-Chlor-6-hydroxy-octansäure zu Verbindungen der Formel (+)-VIII 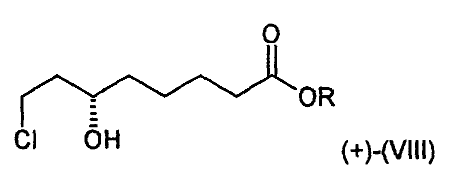 in der R geradkettige und verzweigte C₁ - C₄ -Alkylgruppen bezeichnet, verestert, mit Sulfonsäurechloriden und einer tertiären Stickstoffbase (+)-8-Chlor-6-sulfonyloxy-octansäurealkylester der Formel (+)-II 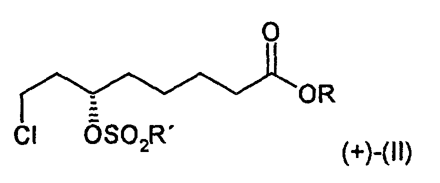 in der R die gleiche Bedeutung wie oben hat und R' geradkettige und verzweigte C₁ - C₄-Alkyl- und Arylgruppen bezeichnet, herstellt und diese mit Alkalimetalldisulfid/Schwefel oder Thioharnstoff zur R-(+)-α-Liponsäure oder (-)-Dihydroliponsäure, welche anschließend durch Oxydation in die R-(+)-α-Liponsäure überführt wird, umsetzt oder
b) (-)-8-Chlor-6-hydroxy-octansäure zu Verbindungen der Formel (-)-VIII 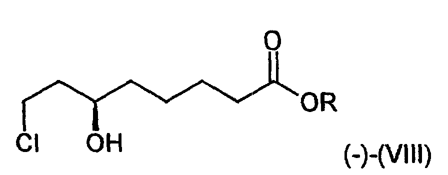 in der R die gleiche Bedeutung wie oben hat, verestert, mit Thionylchlorid in Gegenwart von Pyridin (+)-6,8-Dichlor-octansäurealkylester der Formel (+)-III 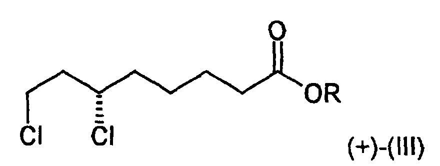 in der R die gleiche Bedeutung wie oben hat, herstellt und diese mit Alkalimetalldisulfid/Schwefel in die R-(+)-α-Liponsäure überführt.

7. Verfahren zur Herstellung von S-(-)-α-Liponsäure, dadurch gekennzeichnet, daß man
a) aus (-)-8-Chlor-6-hydroxy-octansäure mit Sulfonsäurechloriden und einer tertiären Stickstoffbase (-)-8-Chlor-6-sulfonyloxy-octansäure der Formel (-)-I 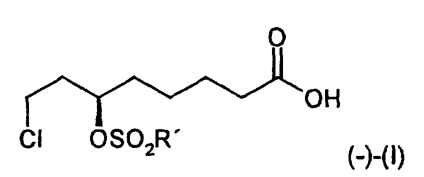 in der R' geradkettige und verzweigte C₁ - C₄-Alkyl- und Arylgruppen bezeichnet, herstellt, diese mit Alkalimetalldisulfid/Schwefel oder Thioharnstoff zur S-(-)-α-Liponsäure oder (+)-Dihydroliponsäure, welche anschließend durch Oxydation in die S-(-)-α-Liponsäure überführt wird, umsetzt oder
b) aus (+)-8-Chlor-6-hydroxy-octansäure mit Thionylchlorid in Gegenwart von Pyridin (-)-6,8-Dichlor-octansäure herstellt und diese mit Alkalimetalldisulfid/Schwefel in die S-(-)-α-Liponsäure überführt.

8. Verfahren zur Herstellung von S-(-)-α-Liponsäure, dadurch gekennzeichnet, daß man
a) (-)-8-Chlor-6-hydroxy-octansäure zu Verbindungen der Formel (-)-VIII 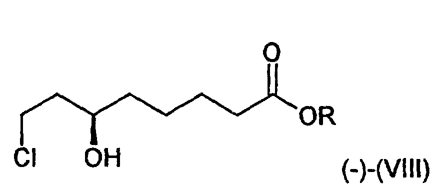 in der R geradkettige und verzweigte C₁ - C₄ -Alkylgruppen bezeichnet, verestert, mit Sulfonsäurechloriden und einer tertiären Stickstoffbase (-)-8-Chlor-6-sulfonyloxyoctansäurealkylester der Formel (-)-II 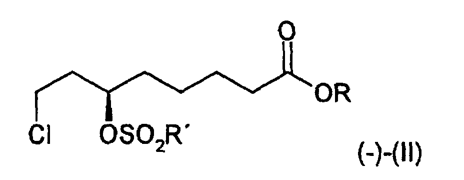 in der R die gleiche Bedeutung wie oben hat und R' geradkettige und verzweigte C₁ - C₄-Alkyl- und Arylgruppen bezeichnet, herstellt und diese mit Alkalimetalldisulfid/Schwefel oder Thioharnstoff zur S-(-)-α-Liponsäure oder (+)-Dihydroliponsäure, welche anschließend durch Oxydation in die S-(-)-α-Liponsäure überführt wird, umsetzt oder
b) (+)-8-Chlor-6-hydroxy-octansäure zu Verbindungen der Formel (+)-VIII 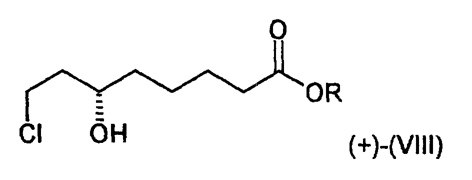 in der R die gleiche Bedeutung wie oben hat, verestert, mit Thionylchlorid in Gegenwart von Pyridin (-)-6,8-Dichlor-octansäurealkylester der Formel (-)-III 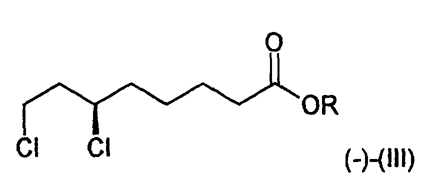 in der R die gleiche Bedeutung wie oben hat, herstellt und diese mit Alkalimetalldisulfid/Schwefel in die S-(-)-α-Liponsäure überführt.

9. Verwendung von (+)- und (-)-8-Chlor-6-sulfonyloxy-octansäuren der Formel 1, 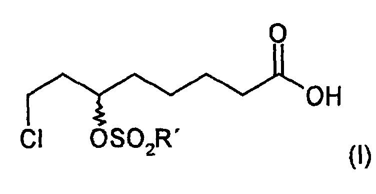 in der R' geradkettige und verzweigte C₁ - C₄-Alkyl- und Arylgruppen bezeichnet, als Zwischenprodukte zur Herstellung von enantiomerenreiner a-Liponsäure.

10. Verwendung von (+)- und (-)-8-Chlor-6-sulfonyloxy-octansäurealkylestern der Formel II, 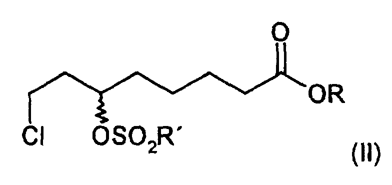 in der R geradkettige und verzweigte C₁ - C₄-Alkylgruppen und R' geradkettige und verzweigte C₁ - C₄-Alkyl- und Arylgruppen bezeichnen, als Zwischenprodukte zur Herstellung von enantiomerenreiner α-Liponsäure.

## Claims

1. (+)- and (-)-8-chloro-6-sulphonyloxyoctanoic acids of the formula I, 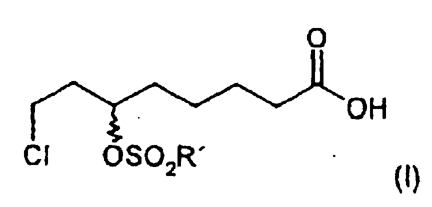 in which R' designates straight-chain and branched C₁--C₄-alkyl and aryl groups.

2. Alkyl (+)- and (-)-8-chloro-6-sulphonyloxyoctanoates of the formula II, 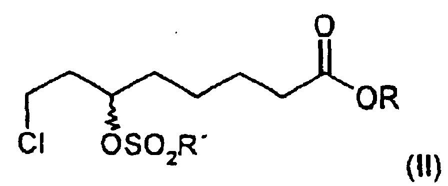 in which R designates straight-chain and branched C₁-C₄-alkyl groups and R' designates straight-chain and branched C₁-C₄-alkyl and aryl groups.

3. Process for the preparation of (+)- and (-)-8-chloro-6-sulphonyloxyoctanoic acids, characterized in that (+)- and (-)-8-chloro-6-hydroxyoctanoic acid are reacted with 1.5 to 3 molar equivalents of sulphonic acid chloride and 1.5 to 2.5 molar equivalents of tertiary nitrogen base at 0 - 30°C in an organic solvent.

4. Process for the preparation of alkyl (+)- and (-)-8-chloro-6-sulphonyloxyoctanoates of the formula II, 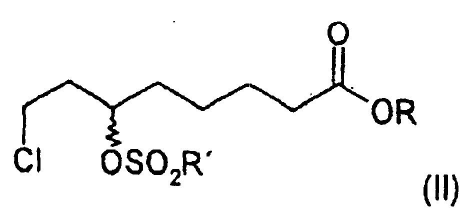 in which R designates straight-chain and branched C₁-C₄ alkyl groups and R' designates straight-chain and branched C₁-C₄-alkyl and aryl groups, characterized in that alkyl (+)- and (-)-8-chloro-6-hydroxyoctanoates of the formula VIII 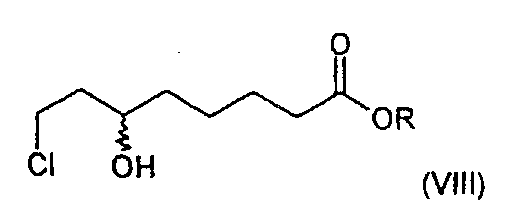 in which R has the same meaning as above, are reacted with 1.0 to 1.5 molar equivalents of sulphonic acid chloride and 1.0 to 1.5 molar equivalents of tertiary nitrogen base at 0 - 30°C in an organic solvent.

5. Process for the preparation of R-(+)-α-lipoic acid, characterized in that
a) starting from (+)-8-chloro-6-hydroxyoctanoic acid with sulphonic acid chlorides and a tertiary nitrogen base, (+)-8-chloro-6-sulphonyloxyoctanoic acid of the formula (+)-I 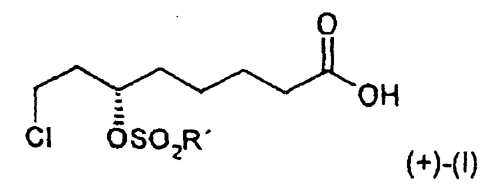 in which R' designates straight-chain and branched C₁-C₄-alkyl and aryl groups, is prepared, this is reacted with alkali metal disulphide/sulphur or thiourea to give R-(+)-α-lipoic acid or (-)-dihydrolipoic acid, which is then converted into R-(+)-α-lipoic acid by oxidation, or
b) starting from (-)-8-chloro-6-hydroxyoctanoic acid with thionyl chloride in the presence of pyridine, (+)-6,8-dichlorooctanoic acid is prepared and this is converted into R-(+)-α-lipoic acid using alkali metal disulphidelsulphur.

6. Process for the preparation of R-(+)-α-lipoic acid, characterized in that
a) (+)-8-chloro-6-hydroxyoctanoic acid is esterified to give compounds of the formula (+)-VIII 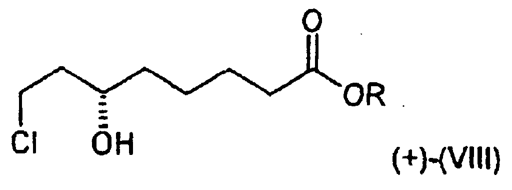 in which R designates straight-chain and branched C₁-C₄-alkyl groups, using sulphonic acid chlorides and a tertiary nitrogen base, alkyl (+)-8-chloro-6-sulphonyloxyoctanoate of the formula (+)-II 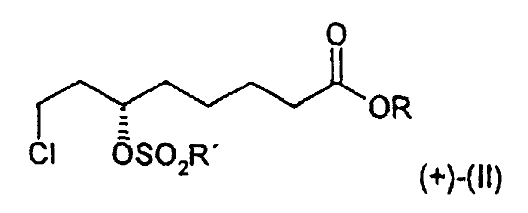 in which R has the same meaning as above and R' designates straight-chain and branched C₁-C₄-alkyl and aryl groups, is prepared and this is reacted with alkali metal disulphide/sulphur or thiourea to give R-(+)-α-lipoic acid or (-)-dihydrolipoic acid, which is then converted into R-(+)-α-lipoic acid by oxidation, or
b) (-)-8-chloro-6-hydroxyoctanoic acid is esterified to give compounds of the formula (-)-VIII 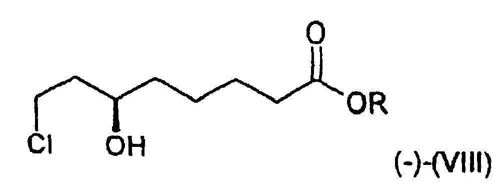 in which R has the same meaning as above, using thionyl chloride in the presence of pyridine, alkyl (+)-6,8-dichlorooctanoate of the formula (+)-III 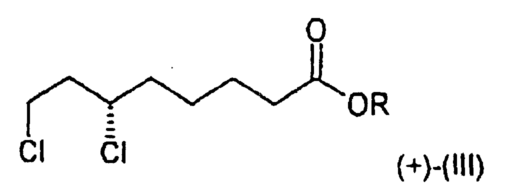 in which R has the same meaning as above, is prepared and this is converted into R-(+)-α-lipoic acid using alkali metal disulphide/sulphur.

7. Process for the preparation of S-(-)-α-lipoic acid, characterized in that
a) starting from (-)-8-chloro-6-hydroxyoctanoic acid using sulphonic acid chlorides and a tertiary nitrogen base, (-)-8-chloro-6-sulphonyloxyoctanoic acid of the formula (-)-I 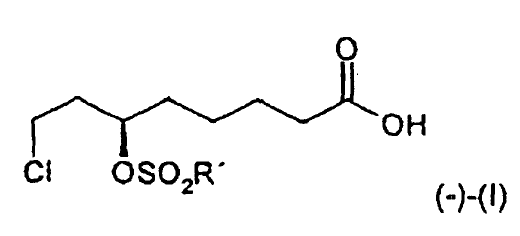 in which R' designates straight-chain and branched C₁-C₄-alkyl and aryl groups, is prepared, this is reacted with alkali metal disulphidelsulphur or thiourea to give S-(-)-α-lipoic acid or (+)-dihydrolipoic acid, which is then converted into S-(-)-α-lipoic acid by oxidation, or
b) starting from (+)-8-chloro-6-hydroxyoctanoic acid using thionyl chloride in the presence of pyridine, (-)-6,8,dichlorooctanoic acid is prepared and this is converted into S-(-)-α-lipoic acid using alkali metal disulphide/sulphur.

8. Process for the preparation of S-(-)-α-lipoic acid, characterized in that
a) (-)-8-chloro-6-hydroxyoctanoic acid is esterified to give compounds of the formula (-)-VIII 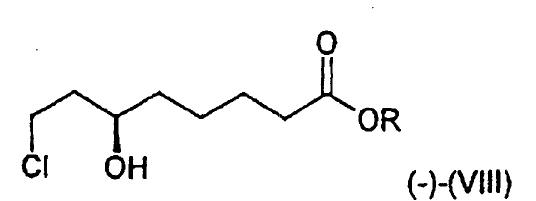 in which R designates straight-chain and branched C₁-C₄-alkyl groups, using sulphonic acid chlorides and a tertiary nitrogen base, alkyl (-)-8-chloro-6-sulphonyloxyoctanoate of the formula (-)-II 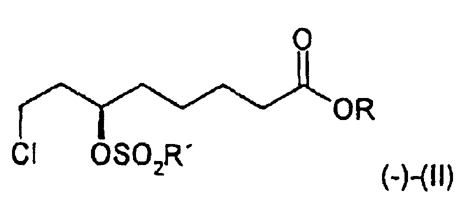 in which R has the same meaning as above and R' designates straight-chain and branched C₁-C₄-alkyl and aryl groups, is prepared and this is reacted with alkali metal disulphide/sulphur or thiourea to give S-(-)-α-lipoic acid or (+)-dihydrolipoic acid, which is then converted into S-(-)-α-lipoic acid by oxidation, or
b) (+)-8-chloro-6-hydroxyoctanoic acid is esterified to give compounds of the formula (+)-VIII 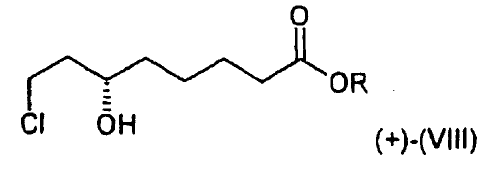 in which R has the same meaning as above, with thionyl chloride in the presence of pyridine, alkyl (-)-6,8-dichlorooctanoate of the formula (-)-III 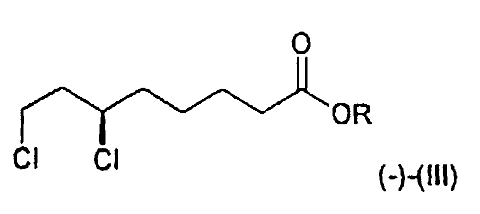 in which R has the same meaning as above, is prepared and this is converted into the S-(-)-α-lipoic acid using alkali metal disulphide/sulphur.

9. Use of alkyl (+)- and (-)-8-chloro-6-sulphonyloxyoctanoic acids of the formula I, 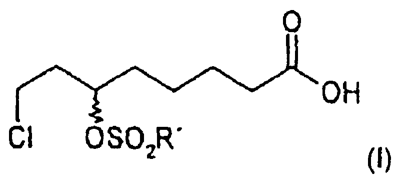 in which R' designates straight-chain and branched C₁-C₄-alkyl and aryl groups, as intermediates for the preparation of enantiomerically pure α-lipoic acid.

10. Use of alkyl (+)- and (-)-8-chloro-6-sulphonyloxyoctanoates of the formula II 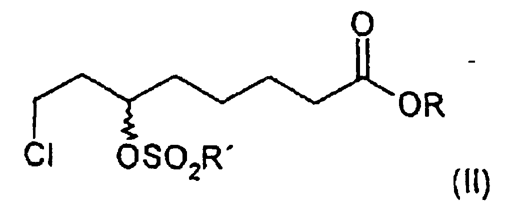 in which R designates straight-chain and branched C₁-C₄-alkyl groups and R' designates straight-chain and branched C₁-C₄-alkyl and aryl groups, as intermediates for the preparation of enantiomerically pure α-lipoic acid.

## Revendications

1. Acides (+)- et (-)-8-chloro-6-sulfonyloxy-octanoïque de formule I 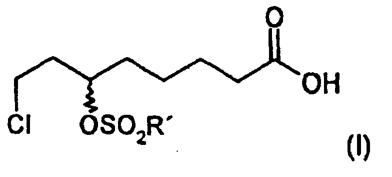 dans laquelle R' représente des groupes alkyle en C1-C4 et aryle à chaîne droite ou ramifiés.

2. Esters alkyliques d'acides (+)- et (-)-8-chloro-6-sulfonyloxy-octanoïque de formule II 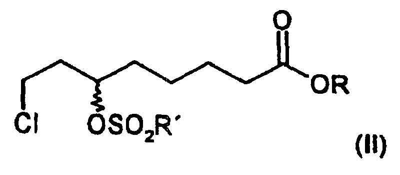 dans laquelle R représente des groupes alkyle en C1-C4 à chaîne droite ou ramifiés et R' représente des groupes alkyle en C1-C4 et aryle à chaîne droite ou ramifiés.

3. Procédé pour la préparation d'acides (+)- et (-)-8-chloro-6-sulfonyloxy-octanoïque, caractérisé en ce que l'on fait réagir des acides (+)- et (-)-8-chloro-6-hydroxyoctanoïque avec 1,5 à 3 équivalents molaires de chlorure de sulfonyle et 1,5 à 2,5 équivalents molaires d'une base azotée tertiaire, à 0-30°C, dans un solvant organique.

4. Procédé pour la préparation d'esters alkyliques d'acides (+)- et (-)-8-chloro-6-sulfonyloxy-octanoïque de formule II 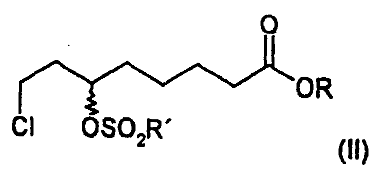 dans laquelle R représente des groupes alkyle en C1-C4 à chaîne droite ou ramifiés et R' représente des groupes alkyle en C1-C4 et aryle à chaîne droite ou ramifiés,
caractérisé en ce qu'
on fait réagir des esters alkyliques d'acides (+)- et (-)-8-chloro-6-hydroxy-octanoïque de formule VIII 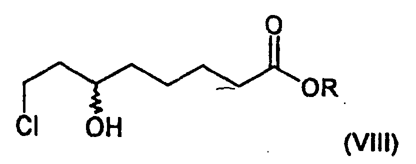 dans laquelle R a la même signification que celle donnée plus haut, avec 1,0 à 1,5 équivalent molaire de chlorure de sulfonyle et 1,0 à 1,5 équivalent molaire d'une base azotée tertiaire, à 0-30°C, dans un solvant organique.

5. Procédé pour la préparation d'acide R-(+)-α-lipoïque,
caractérisé en ce qu'
a) à partir d'acide (+)-8-chloro-6-hydroxy-octanoïque, avec des chlorures de sulfonyle et une base azotée tertiaire, on prépare un acide (+)-8-chloro-6-sulfonyloxy-octanoïque de formule (+)-I 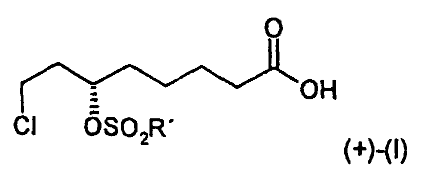 dans laquelle R' représente des groupes alkyle en C1-C4 et aryle à chaîne droite ou ramifiés, on fait réagir ce dernier avec du soufre/disulfure de métal alcalin ou de la thio-urée, pour obtenir l'acide R-(+)-α-lipoïque ou l'acide (-)-dihydrolipoïque qui est ensuite converti par oxydation en l'acide R-(+)-α-lipoïque, ou
b) à partir d'acide 8-chloro-6-hydroxy-octanoique, avec du chlorure de thionyle en présence de pyridine, on prépare un acide (+)-6,8-dichloro-octanoïque, et on convertit ce dernier, avec du soufre/disulfure de métal alcalin, en l'acide R-(+)-α-lipoïque.

6. Procédé pour la préparation d'acide R-(+)-α-lipoïque,
caractérisé en ce qu'
a) on estérifie de l'acide (+)-8-chloro-6-hydroxyoctanoïque pour aboutir aux composés de formule (+)-VIII 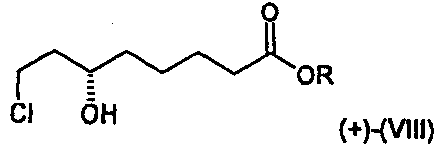 dans laquelle R représente des groupes alkyle en C1-C4 à chaîne droite ou ramifiés, on prépare avec des chlorures de sulfonyle et une base azotée tertiaire un ester alkylique d'acide (+)-8-chloro-6-sulfonyloxy-octanoïque de formule (+)-II 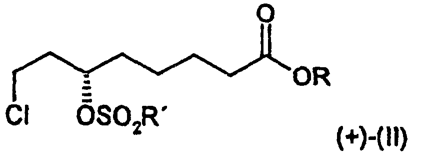 dans laquelle R a la même signification que celle donnée plus haut et R' représente des groupes alkyle en C1-C4 et aryle à chaîne droite ou ramifiés, et on fait réagir ce dernier avec du soufre/disulfure de métal alcalin ou de la thio-urée, pour aboutir à l'acide R-(+)-α-lipoïque ou à l'acide (-)-dihydrolipoïque qui est ensuite converti par oxydation en l'acide R-(+)-α-lipoïque, ou
b) on estérifie de l'acide (-)-8-chloro-6-hydroxyoctanoïque pour aboutir aux composés de formule (-)-VIII 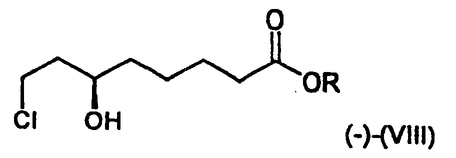 dans laquelle R a la même signification que celle donnée plus haut, on prépare avec du chlorure de thionyle, en présence de pyridine, un ester alkylique d'acide (+)-6,8-dichloro-octanoïque de formule (+)-III 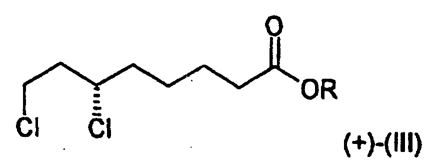 dans laquelle R a la même signification que celle donnée plus haut, et on convertit ce dernier, avec du soufre/ disulfure de métal alcalin, en l'acide R-(+)-α-lipoïque.

7. Procédé pour la préparation d'acide S-(-)-α-lipoïque,
caractérisé en ce qu'
a) à partir d'acide (-)-8-chloro-6-hydroxy-octanoïque, avec des chlorures de sulfonyle et une base azotée tertiaire, on prépare un acide (-)-8-chloro-6-sulfonyloctanoïque de formule (-)-I 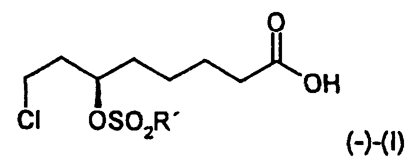 dans laquelle R' représente des groupes alkyle en C1-C4 et aryle à chaîne droite ou ramifiés, on fait réagir ce dernier avec du soufre/disulfure de métal alcalin ou de la thio-urée, pour aboutir à l'acide S-(-)-α-lipoïque, ou à l'acide (+)-dihydrolipoïque qui est ensuite converti par oxydation en l'acide S-(-)-α-lipoïque,
b) à partir d'acide (+)-8-chloro-6-hydroxy-octanoïque, avec du chlorure de thionyle en présence de pyridine, on prépare de l'acide (-)-6,8-dichloro-octanoïque, et on convertit ce dernier, avec du soufre/disulfure de métal alcalin, en l'acide S-(-)-α-lipoïque.

8. Procédé pour la préparation d'acide S-(-)-α-lipoïque,
caractérisé en ce qu'
a) on estérifie de l'acide (-)-8-chloro-6-hydroxyoctanoïque pour aboutir aux composés de formule (-)-VIII 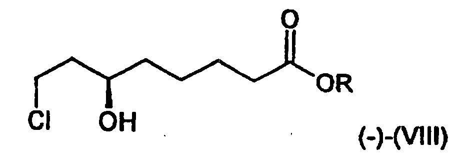 dans laquelle R représente des groupes alkyle en C1-C4 à chaîne droite ou ramifiés, on prépare avec des chlorures de sulfonyle et une base azotée tertiaire des esters alkyliques d'acide (-)-8-chloro-6-sulfonyloxy-octanoïque de formule (-)-II 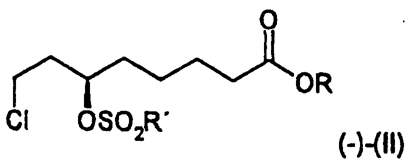 dans laquelle R a la même signification que celle donnée plus haut et R' représente des groupes alkyle en C1-C4 et aryle à chaîne droite ou ramifiés, et on fait réagir ces derniers avec du soufre/disulfure de métal alcalin ou de la thio-urée, pour aboutir à l'acide S-(-)-α-lipoïque ou à l'acide (+)-dihydrolipoïque qui est ensuite converti par oxydation en l'acide S-(-)-α-lipoïque, ou
b) on estérifie de l'acide (+)-8-chloro-6-hydroxyoctanoïque pour aboutir aux composés de formule (+)-VIII 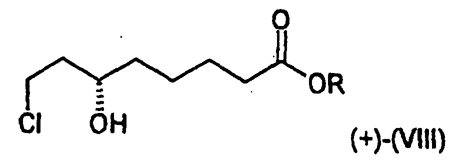 dans laquelle R a la même signification que celle donnée plus haut, on prépare avec du chlorure de thionyle, en présence de pyridine, des esters alkyliques d'acide (-)-6,8-dichloro-octanoïque de formule (-)-III 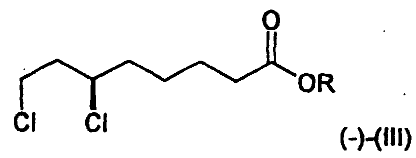 dans laquelle R a la même signification que celle donnée plus haut, et on convertit ces derniers, avec du soufre/disulfure de métal alcalin, en l'acide S-(-)-α-lipoïque.

9. Utilisation des acides (+)- et (-)-8-chloro-6-sulfonyloxy-octanoïque de formule I 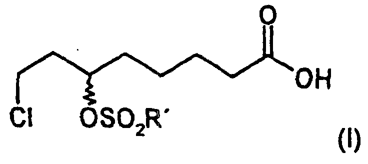 dans laquelle R' représente des groupes alkyle en C1-C4 et aryle à chaîne droite ou ramifiés, en tant que produits intermédiaires pour la préparation d'acide α-lipoïque sous forme d'énantiomère pur.

10. Utilisation d'esters alkyliques d'acide (+)- et (-)-8-chloro-6-sulfonyloxy-octanoïque de formule II 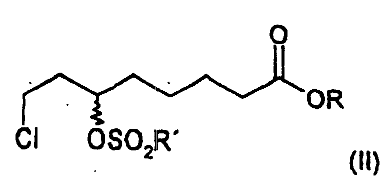 dans laquelle R représente des groupes alkyle en C1-C4 à chaîne droite ou ramifiés, et R' représente des groupes alkyle en C1-C4 et aryle à chaîne droite ou ramifiés, en tant que produits intermédiaires pour la préparation d'acide a-lipoïque sous forme d'énantiomère pur.
